Europäisches Patentamt

European Patent Office

Office européen des brevets

(11) Publication number : **0 437 114 B1**

(12)

# EUROPEAN PATENT SPECIFICATION

(45) Date of publication of patent specification :
23.03.94 Bulletin 94/12

(51) Int. Cl.⁵ : **A61K 7/075**

(21) Application number : **90314451.7**

(22) Date of filing : **31.12.90**

(54) **Shampoo composition.**

The file contains technical information
submitted after the application was filed and
not included in this specification

(30) Priority : **10.01.90 GB 9000237**

(43) Date of publication of application :
**17.07.91 Bulletin 91/29**

(45) Publication of the grant of the patent :
**23.03.94 Bulletin 94/12**

(84) Designated Contracting States :
**AT BE CH DE DK ES FR GB GR IT LI NL SE**

(56) References cited :
**EP-A- 0 150 323
EP-A- 0 218 931
FR-A- 2 534 473
GB-A- 1 470 234
US-A- 4 061 602
US-A- 4 772 462
G.A. NOVAK "DIE KOSMETISCHEN PR PARA-
TE", vol. 2, 1984, Verlag fÜr chemische Indus-
trie, H. Ziolkowsky KG, Augsburg**

(73) Proprietor : **UNILEVER PLC
Unilever House Blackfriars P.O. Box 68
London EC4P 4BQ (GB)**
(84) **GB**
Proprietor : **UNILEVER N.V.
Weena 455
NL-3013 AL Rotterdam (NL)**
(84) **BE CH DE DK ES FR GR IT LI NL SE AT**

(72) Inventor : **Gallagher, Peter, c/o
Chesebrough-Ponds Inc.
150 Bullock Drive
Markham, Ontario, L3P 1W3 (CA)**
Inventor : **Walmsley, Patricia Jane
3 Banks Road
Heswall, Wirral, Merseyside (CA)**
Inventor : **McGee, Tom
Roure, BP. 24, 55 Voie des Barrs
F-95102 Argenteuil Cedex (FR)**

(74) Representative : **Barker, Rosemary Anne et al
c/o Mewburn Ellis 2 Cursitor Street
London EC4A 1BQ (GB)**

EP 0 437 114 B1

## Description

### Field of the Invention

The present invention relates to an aqueous shampoo composition, and more particularly to an aqueous shampoo composition containing carboxylic acid and a film-forming polymer.

### Background of the Invention

The use of citric acid in hair compositions has long been known, for example, US 2 255 341 refers to a "lemon" hair rinse, used to restore an acid pH, following the more alkaline shampoo treatment generally used in the 1940's.

Citric acid has also been used as a pH regulator in hair treatment compositions, for example in JP 74 21070 (Tanabe), and in US 4 752 467 (Wella) a synergistic combination of citric acid and betaine with formation of a betaine salt is said to improve combability of the hair.

The use of film-forming polymers in hair conditioning shampoos is known for example from US-A-4 061 602 and EP-A-0 218 931.

Attempts have been made to improve the set and curl retention of hair by introducing film-forming polymers into shampoos.

Certain of these film-forming polymers have been shown to give some improvement to manageability and curl retention. For example US 3 400 198 (Procter & Gamble) relates to the use of polyethyleneimine in shampoos, and in GB 1 470 234 (Union Carbide), certain water-soluble quaternary nitrogen containing cellulose ethers in combination with amphoteric or nonionic surfactants are said to improve the condition and set of hair from a shampoo.

We have now surprisingly found that the set and style retention obtained from a shampoo can be greatly improved by the use of a shampoo which comprises a film-forming polymer together with a relatively high level of carboxylic acid, the pH of the composition being from 2 to 5.

The film-forming properties of the polymer are enhanced on the hair by the presence of the carboxylic acid, thereby avoiding the need for high levels of these expensive ingredients.

### Brief summary of the Invention

Accordingly, the invention provides an aqueous shampoo composition comprising
(a) from 0.5 to 40% by weight of at least one anionic surfactant;
(b) from 0.01 to 10% by weight of film-forming polymer or mixtures thereof; and
(c) from 2.5 to 20% by weight of aryl or $C_{1-18}$ alkyl carboxylic acid, $C_{1-12}$ alkyl or aryl derivatives or mixtures thereof,
the composition having a pH of from 2 to 5.

The invention further comprises a method for improving the curl retention of hair which comprises the step of treating the hair with the compositions of the invention.

### Detailed Description of the Invention

#### (a) Anionic surfactant

The composition of the invention comprises anionic surfactant.

Suitable anionic surfactants include alkyl sulphates, alkyl ether sulphates, alkyl benzene sulphonates, alpha-olefin sulphonates, acyl glutamates, acyl peptides, alkyl sarcosinates, alkyl taurates and alkyl sulphosuccinates.

The pH of the composition lies in the range of from 2 to 5, preferably from 2.5 to 3.5, and at the lower end of this range it is preferred that the composition comprises an anionic surfactant which is especially stable at acid pH. Examples of such acid-stable anionic surfactants include alkyl benzene sulphonates and α-olefin sulphonates.

The composition of the invention comprises from 0.5 to 40% by weight of anionic surfactant, preferably from 6 to 18% by weight. If an amount of less than 0.5% by weight of anionic surfactant is present, inadequate foaming is achieved, and if more than 40% by weight is present, no further increase in cleansing power or foaming ability is likely to be observed.

### (b) Film-forming polymer

The composition of the invention comprises film-forming polymer or mixtures thereof, which impart to the hair improved body and enhanced set and style retention. The degree of set and style retention can be assessed by measuring curl strength ie. the durability of a curl formed on treated hair, and we will demonstrate in the Comparative Examples that the composition of the invention which comprises film-forming polymer together with carboxylic acid gives rise to a more durable curl than hair treated with compositions comprising film-forming polymer alone.

The compositions of the invention therefore have improved set and style retention, or may comprise lesser amounts of the expensive film-forming polymers to achieve the same level of curl retention as previously known compositions.

The film-forming polymers are preferably chosen from Quaternium 19, available commercially as Polymer JR (Union Carbide), Quaternium 23, available commercially as Gafquat® 755 (GAF Corporation), Quaternium 41, available commercially as Merquat® 550 (Merck), chitosan or derivatives thereof steardimonium hydrolysed animal collagen, available commercially as Croquat® S, lauryl dimonium hydrolysed animal protein, available commercially as Lamequat® L or methyl vinyl imidazolium chloride/vinyl pyrrolidone copolymer.

The composition of the invention comprises from 0.01 to 10% by weight of the film-forming polymer, preferably from 0.1 to 5% by weight. If less than 0.01% by weight is present, little curl retention benefit is observed, and if the composition comprises more than 10% by weight, little increase in benefit is seen, and the hair may appear dull, lifeless and cosmetically unappealing.

### (c) Carboxylic acid or derivatives thereof

The composition of the invention also comprises a $C_{1-18}$ carboxylic acid, or $C_{1-12}$ alkyl or aryl derivative or mixtures thereof. The acid may be mono-, di- or tri-basic and is preferably a hydroxycarboxylic acid. Suitable examples of acids which may be used in compositions of the invention include lactic, tartaric, benzoic, hydroxybenzoic, hydroxyoctanoic and gallic acids. The acid is preferably citric acid.

According to the invention the carboxylic acid derivatives are $C_{1-12}$ alkyl or aryl derivatives, for example methyl citrate and dodecyl citrate. Mixtures of carboxylic acids and symmetrical/unsymmetrical derivatives thereof are also within the scope of the invention.

The composition of the invention comprises from 2.5 to 20% of the said carboxylic acid, derivatives or mixtures thereof. Preferably, the composition comprises from 5 to 10% by weight of $C_{1-18}$ carboxylic acid, $C_{1-12}$ alkyl or aryl derivatives or mixtures thereof.

At levels of less than 2.5% by weight of carboxylic acid or its derivative, little enhancement of the effect of the film-forming polymer is observed, and if the composition comprises more than 20% by weight of the acid, the pH falls to a physiologically unacceptable level. It may be necessary to adjust the pH using alkali such as sodium hydroxide or triethanolamine.

The pH of the shampoo composition lies between 2 and 5, and is preferably from 2.5 to 3.5. Little increase in set and style retention is seen at a pH of greater than 5.

### Other ingredients

The shampoo composition of the invention may further comprise certain ingredients known in the art and necessary to the particular formulation. Examples of other ingredients include other surfactants, eg. amphoteric such as betaines, viscosity control agents, solubility control agents, foam boosters, opacifiers, perfumes, colouring agents, conditioning agents, pearlescers, preservatives, proteins and buffering agents.

### COMPARATIVE EXAMPLES

The following comparative examples illustrate the effect of carboxylic acid and film-forming polymers on the set and style retention of the hair.

### Comparative-Example 1

### Measurement of curl strength

A 4g/20 cm switch of Yugoslavian red tie hair is wet, shampooed twice with 0.25 ml of sample for 30 sec-

EP 0 437 114 B1

onds, and rinsed under running tap water at 40°C for 30 seconds. Excess water is squeezed out, and the switch is mounted on 30 mm diameter rollers, dried at 50°C for 1 hour and equilibrated overnight at 70°C/50% relative humidity.

The curl strength is measured using an Instron Tensile Tester, by placing the switch between fixed bars and determining the force in grams required to uncurl the switch.

The following samples were tested:

| | % wt | | | |
|---|---|---|---|---|
| | Control | A | B | C |
| SLES 2EO | 13.0 | 13.0 | 13.0 | 13.0 |
| Tegobetaine® L7* | 6.0 | 6.0 | 6.0 | 6.0 |
| Sodium chloride | 1.5 | 1.5 | 1.5 | 1.5 |
| Croquat® S | – | 0.3 | – | 0.3 |
| Citric acid | – | – | 10.0 | 10.0 |
| Luviquat® | – | – | 1.0 | – |
| Water to | 100 | 100 | 100 | 100 |
| | | | | |
| Curl strength (g) | 7.9 | 9.74 | 10.53 | 12.50 |

\* Tegobetaine® L7 is cocamidopropyl betaine

The pH was adjusted to 3.5 with sodium hydroxide.

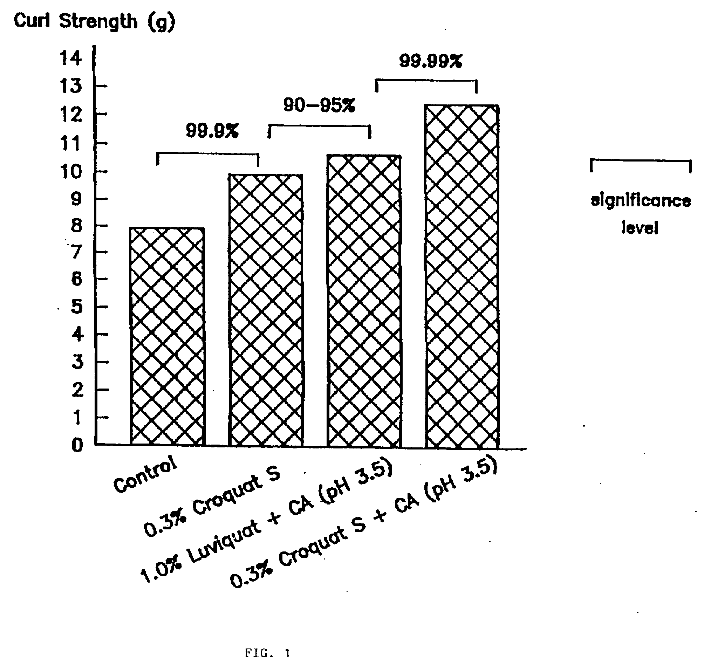

Results of the curl strength assessment are shown on Figure 1, and it can be seen that curl strength is improved over the control sample by adding film-forming polymer alone (Sample A), and an even greater improvement over the control is seen when both film-forming polymer and citric acid are present in the sample (Samples B and C).

Comparative Example 2

The effect of adding citric acid to a shampoo composition containing 0.5% by weight of Polymer® JR 400 as film-forming polymer was assessed.

| | %wt | | | |
|---|---|---|---|---|
| Sample | D | E | F | G |
| SLES 2EO | 12.0 | 12.0 | 12.0 | 12.0 |
| Sodium chloride | 2.75 | 2.75 | 2.75 | 2.75 |
| Citric acid | 0 | 2.5 | 5 | 10 |
| Polymer® JR 400 | 0.5 | 0.5 | 0.5 | 0.5 |
| Water to | 100 | 100 | 100 | 100 |
| | | | | |
| Curl strength (g) | 10.5 | 10.6 | 14.7 | 19.2 |

4

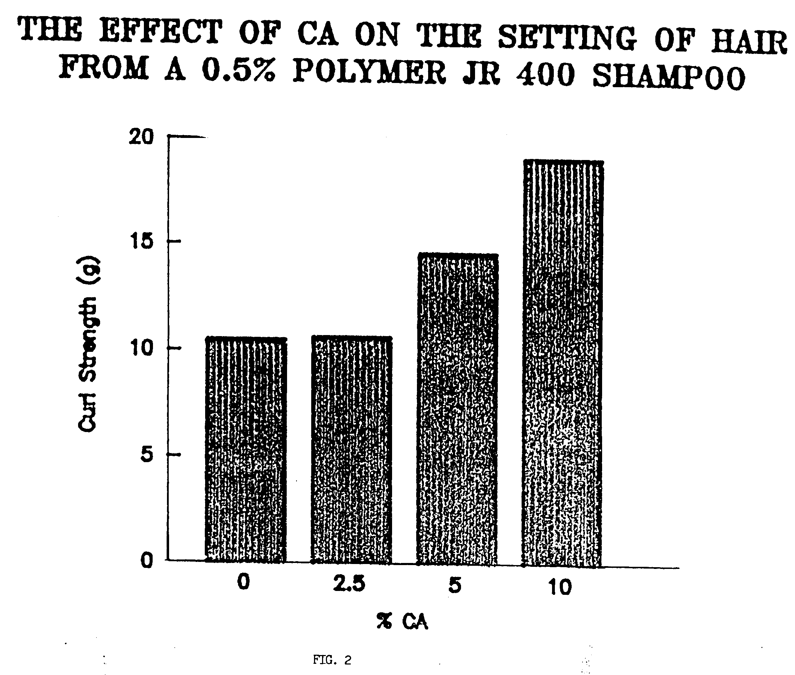

Curl strength of switches treated with samples D, E, F and G were assesed using the method outlined in Comparative Example 1, and the results are presented in Figure 2.

It can be seen that addition of citric acid to a shampoo containing Polymer® JR gives curls of increasing strength.

Comparative Example 3

The effect of pH on the setting of hair was assessed when using a shampoo containing 10% by weight citric acid and 0.5% by weight Polymer® JR 400.

Sample shampoos were prepared by mixing 12% by weight sodium lauryl sulphate, 10% by weight citric acid and 0.5% by weight Polymer® JR 400. The pH of the samples was adjusted to 3.5, 4.5 and 6.0 respectively by addition of sodium hydroxide.

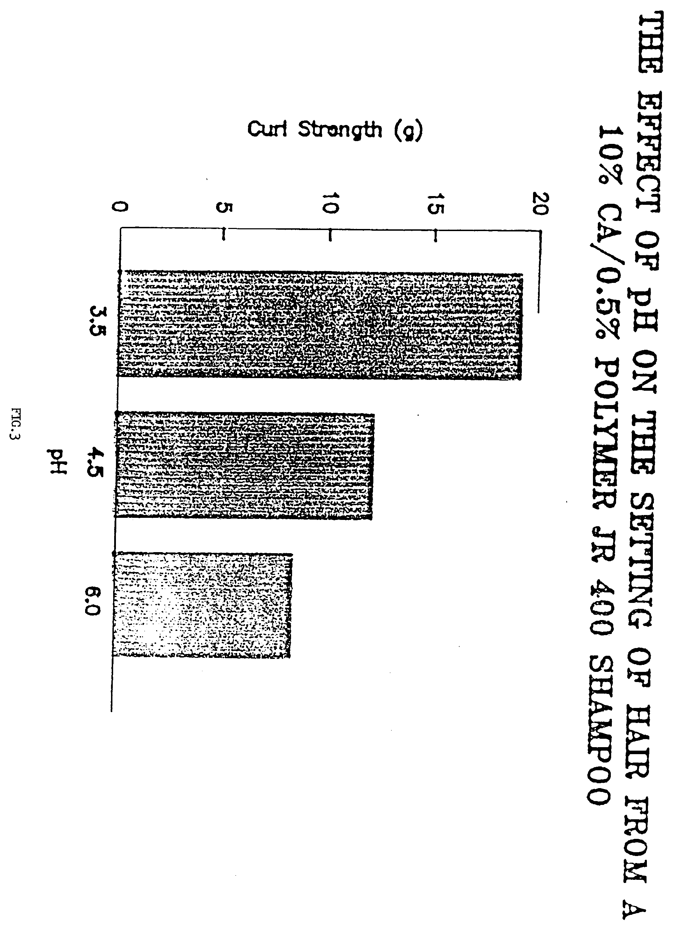

Curl strength of switches treated with these samples was assessed using the method outlined in Comparative Example 1, and the results are presented in Figure 3.

The sample shampoos gave curl strengths of 19.2g, 12.1g and 8.4g respectively.

The Figure shows that as the pH increases the curl strength decreases. At a pH of above 5, the curl strength is found to drop below 10g which is the curl strength obtained by treating a switch with a shampoo containng no citric acid (see Fig. 2).

The invention is further illustrated by the following examples.

EXAMPLES

Example 1

The following ingredients were mixed together to form a shampoo composition:

|                                   | % wt   |
|-----------------------------------|--------|
| Sodium lauryl sulphate            | 12     |
| Polymer® JR                       | 0.2    |
| Citric Acid                       | 10.0   |
| Bronopol*                         | 0.01   |
| Butyl hydroxy toluene (BHT)       | 0.05   |
| Colour, perfume                   | qs     |
| Water                             | to 100 |

The pH was adjusted to 4.5 with sodium hydroxide.

    * Bronopol is 2-bromo-nitropropane-1,3-diol

Example 2

The following ingredients were mixed together to form a shampoo composition:

EP 0 437 114 B1

|  | % wt |
|---|---|
| $\alpha$-olefin sulphonate | 9.0 |
| Citric acid | 10.0 |
| Luviquat® FC 550 [1] | 1.0 |
| Glucamate® DOE 120 [2] | 4.0 |
| Bronopol | 0.01 |
| BHT | 0.05 |
| Colour, perfume | qs |
| Water | to 100 |

The pH was adjusted to 3.5 with sodium hydroxide.

1 - Luviquat® FC 550 is methyl vinyl imidazolium chloride/vinyl pyrrolidone copolymer.

2 - Glucamate® DOE 120 has the CTFA designation PEG-120 methyl glucose dioleate, and is present in the composition as a thickener.

Example 3

The following ingredients were mixed together to form a shampoo composition:

|  | % wt |
|---|---|
| Alkyl benzene sulphonate | 10.0 |
| Acetic acid salt of chitosan | 0.5 |
| Citric acid | 5.0 |
| Bronopol | 0.01 |
| BHT | 0.05 |
| Colour, perfume | qs |
| Water | to 100 |

The pH was adjusted to 3.5 with sodium hydroxide.

Example 4

The following ingredients were mixed together to form a shampoo composition.

6

|  | %wt |
|---|---|
| Alkyl benzene sulphonate | 13.0 |
| Polymer® JR | 0.25 |
| Lactic acid | 8.0 |
| Bronopol | 0.01 |
| BHT | 0.05 |
| Colour, perfume | q.s. |
| Water | to 100 |

The pH was adjusted to 3.7 with sodium hydroxide.

Example 5

The following ingredients were mixed together to form a shampoo composition:

|  | %wt |
|---|---|
| ∝-olefin sulphonate | 9 |
| Tartaric acid | 9 |
| Luviquat® FC 550 | 1.5 |
| Glucamate® DOE 120 | 4.5 |
| Bronopol | 0.01 |
| BHT | 0.05 |
| Colour, perfume | q.s. |
| Water | to 100 |

The pH was adjusted to 4.0 with sodium hydroxide.

Example 6

The following ingredients were mixed together to form a shampoo composition:

|  | %wt |
|---|---|
| SLES 2EO | 12.0 |
| Polymer® JR | 0.35 |
| Citric acid | 6.0 |
| Bronopol | 0.01 |
| BHT | 0.05 |
| Colour, perfume | q.s. |
| Water | to 100 |

The pH was adjusted to 3.4 with sodium hydroxide.

7

## Claims

1. An aqueous shampoo composition comprising
    (a) from 0.5 to 40% by weight of at least one anionic surfactant,
    (b) from 0.01 to 10% by weight of a film-forming polymer or mixtures thereof, and
    (c) from 2.5 to 20% by weight of aryl or $C_{1-18}$ alkyl carboxylic acid, $C_{1-12}$ alkyl or aryl derivatives or mixtures thereof,
    the composition having a pH of from 2 to 5.

2. An aqueous shampoo composition as claimed in Claim 1 wherein the anionic surfactant is chosen from alkyl sulphates, alkyl ether sulphate, alkyl benzene sulphonates, alpha-olefin sulphonates, acyl glutamates, acyl peptides, alkyl sarcosinates, alkyl taurates and alkyl sulphosuccinates.

3. An aqueous shampoo composition as claimed in Claims 1 or 2 which comprises from 6 to 18% by weight of anionic surfactant.

4. An aqueous shampoo composition as claimed in any preceding claim wherein the film-forming polymer is chosen from Quaternium 19, Quaternium 23, Quaternium 41, chitosan or derivatives thereof, steardimonium hydrolysed collagen, lauryl dimonium hydrolysed animal protein or methyl vinyl imidazolium chloride/vinyl pyrrolidone copolymer.

5. An aqueous shampoo composition as claimed in any preceding claim which comprises from 0.1 to 5% by weight of film-forming polymer.

6. An aqueous shampoo composition as claimed in any preceding claim which comprises from 5 to 10% by weight of $C_{1-18}$ carboxylic acid, $C_{1-12}$ alkyl or aryl derivatives or mixtures thereof.

7. An aqueous shampoo composition as claimed in any preceding claim wherein the aryl or $C_{1-18}$ alkyl carboxylic acid is chosen from citric, lactic, tartaric, benzoic, hydroxybenzoic, hydroxyoctanoic or gallic acids.

8. An aqueous shampoo composition as claimed in any preceding claim wherein the pH of the composition is from 2.5 to 3.5.

9. A method for improving the curl retention of hair which comprises the step of treating the hair with a composition according to any of Claims 1 to 8.

## Patentansprüche

1. Wässerige Shampoozusammensetzung, enthaltend
    (a) von 0,5 bis 40 Gewichtsprozent von zumindest einem anionischen Surfactant,
    (b) von 0,01 bis 10 Gewichtsprozent eines filmbildenden Polymeren oder Mischungen derselben, und
    (c) von 2,5 bis 20 Gewichtsprozent Aryl- oder $C_{1-18}$-Alkylcarbonsäure, $C_{1-12}$-Alkyl- oder Arylderivate, oder Mischungen derselben,
    wobei die Zusammensetzung einen pH-Wert von 2 bis 5 aufweist.

2. Wässerige Shampoozusammensetzung nach Anspruch 1, worin das anionische Surfactant aus Alkylsulfaten, Alkylethersulfat, Alkylbenzolsulfonaten, α-Olefinsulfonaten, Acylglutamaten, Acylpeptiden, Alkylsarcosinaten, Alkyltauraten und Alkylsulfosuccinaten ausgewählt ist.

3. Wässerige Shampoozusammensetzung nach einem der Ansprüche 1 oder 2, die von 6 bis 18 Gewichtsprozent anionisches Surfactant enthält.

4. Wässerige Shampoozusammensetzung nach einem der vorhergehenden Ansprüche, worin das filmbildende Polymere aus Quaternium 19, Quaternium 23, Quaternium 41, Chitosan oder Derivaten davon, Steardimonium-hydrolysiertes Kollagen, Lauryldimoniumhydrolysiertes tierisches Protein oder Methylvinylimidazoliumchlorid/Vinylpyrrolidon-Copolymeres ausgewählt ist.

5. Wässerige Shampoozusammensetzung nach einem der Ansprüche 1 bis 4, die von 0,1 bis 5 Gewichtsprozent des filmbildenden Polymeren enthält.

6. Wässerige Shampoozusammensetzung nach einem der Ansprüche 1 bis 5, die von 5 bis 10 Gewichtsprozent $C_{1-18}$-Carbonsäure, $C_{1-12}$-Alkyl- oder Arylderivate, oder Mischungen derselben, enthält.

7. Wässerige Shampoozusammensetzung nach einem der Ansprüche 1 bis 6, worin die Aryl- oder $C_{1-18}$-Alkylcarbonsäure aus Citronensäure, Milchsäure, Weinsäure, Benzoesäure, Hydroxybenzoesäure, Hydroxyoctansäure oder Gallussäuren ausgewählt ist.

8. Wässerige Shampoozusammensetzung nach einem der Ansprüche 1 bis 7, worin der pH-Wert der Zusammensetzung im Bereich von 2,5 bis 3,5 liegt.

9. Verfahren zur Verbesserung der Lockenerhaltung von Haar, welches die Stufe des Behandelns des Haars mit einer Zusammensetzung gemäß irgendeinem der Ansprüche 1 bis 8 umfaßt.

**Revendications**

1. Une composition aqueuse sous forme de shampooing comprenant
   (a) de 0,5 à 40% en masse d'au moins un agent tensioactif anionique;
   (b) de 0,01 à 10% en masse de polymère formant un film ou de mélanges de ceux-ci ; et
   (c) de 2,5 à 20% en masse d'acide carboxylique aryle ou alkyle en $C_{1-18}$, de dérivés alkyle ou aryle en $C_{1-12}$, ou de mélanges de ceux-ci,
   la composition ayant un pH allant de 2 à 5.

2. Une composition aqueuse sous forme de shampooing selon la revendication 1, dans laquelle l'agent tensioactif anionique est choisi à partir d'alkyl sulphates, d'alkyl éther sulphates, d'alkyl benzène sulphonates, d'alpha-oléfines sulphonates, d'acyl glutamates, d'acyl peptides, d'alkyl sarcosinates, d'alkyl taurates et d'alkyl sulphosuccinates.

3. Une composition aqueuse sous forme de shampooing selon l'une des revendications 1 ou 2, comprenant de 6 à 18% en masse d'agent tensioactif anionique.

4. Une composition aqueuse sous forme de shampooing selon l'une des revendications précédentes, dans laquelle le polymère formant un film est choisi à partir du Quaternium 19, du Quaternium 23, du Quaternium 41, de chitosane ou des dérivés de celle-ci, de collagène animal hydrolysé de stéardimonium, de protéine animale hydrolysée de lauryl dimonium, ou de copolymère de chlorure de méthyl vinyl imidazolium/vinyl pyrrolidone.

5. Une composition aqueuse sous forme de shampooing selon l'une des revendications précédentes, comprenant de 0,1 à 5% en masse de polymère formant un film.

6. Une composition aqueuse sous forme de shampooing selon l'une des revendications précédentes, comprenant de 5 à 10% en masse d'acide carboxylique en $C_{1-18}$, de dérivés alkyles ou aryles en $C_{1-12}$ ou de mélanges de ceux-ci.

7. Une composition aqueuse sous forme de shampooing selon l'une des revendications précédentes, dans laquelle l'acide carboxylique aryle ou alkyle en $C_{1-18}$ est choisi à partir d'acide citrique, lactique, tartarique, benzoïque, hydroxybenzoïque, hydroxyoctanoïque ou gallique.

8. Une composition aqueuse sous forme de shampooing selon l'une des revendications précédentes, dans laquelle le pH de la composition va de 2,5 à 3,5.

9. Un procédé pour améliorer le maintien des boucles des cheveux comprenant l'étape de traitement des cheveux avec une composition en accord avec l'une des Revendications 1 à 8.

FIG. 1

# THE EFFECT OF CA ON THE SETTING OF HAIR
## FROM A 0.5% POLYMER JR 400 SHAMPOO

FIG. 2

EP 0 437 114 B1

# THE EFFECT OF pH ON THE SETTING OF HAIR FROM A 10% CA/0.5% POLYMER JR 400 SHAMPOO

FIG.3

EP 0 437 114 B1

12